(19) Europäisches Patentamt
European Patent Office
Office européen des brevets

(11) Publication number: 0 028 269
B1

(12) # EUROPEAN PATENT SPECIFICATION

(45) Date of publication of patent specification: 19.05.82

(51) Int. Cl.³: C 07 D 213/30, A 01 N 43/40

(21) Application number: 79104290.6

(22) Date of filing: 02.11.79

(54) **Pyridylalkyl esters, insect repellent compositions and method of repelling insects.**

(43) Date of publication of application:
13.05.81 Bulletin 81/19

(45) Publication of the grant of the patent:
19.05.82 Bulletin 82/20

(84) Designated Contracting States:
AT BE CH DE FR GB IT NL SE

(56) References cited:
US - A - 2 524 838
US - A - 3 438 993
US - A - 3 637 714

(73) Proprietor: STAUFFER CHEMICAL COMPANY
Westport Connecticut 06880 (US)

(72) Inventor: Young Wong, Rayman
3411 Lowell Avenue
Richmond, Cal. 94804 (US)

(74) Representative: Jaeger, Klaus, Dr.rer.nat. Dipl.-Chem. et al,
Jaeger, Grams & Pontani Patentanwälte
Bergstrasse 48 1/2
D-8035 München-Gauting (DE)

EP 0 028 269 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Pyridylalkyl esters, insect repellant compositions and method of repelling insects.

## Background of the invention

This invention relates to novel compounds having the formula

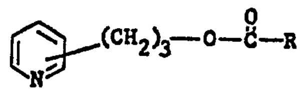

in which R is $C_3$—$C_5$ alkyl or $C_3$—$C_5$ alkenyl.

Some compounds of similar structure are already known in the art. However, these compounds are prepared from pyridine methanol or pyridine ethanol rather than pyridine propanol, or alternatively, have a substituent corresponding to the group R but having fewer than 3 three carbon atoms. In addition, the compounds in the prior art are not indicated as possessing any insect repellent properties.

Thus, for instance, esters of pyridine methanol and pyridine ethanol are disclosed in U.S. Patents 2,524,838, 3,428,993, and 3,637,714 as well as journal such articles as Boekelheide et al., *Journal of the American Chemical Society,* Vol. 76, pp. 1286—1291 (1954); Lal et al., *Journal of Organic Chemistry,* Vol. 20, pp. 1030—1033 (1955); Ito et al., *Polymer Journal* (Japan), Vol. 1, No. 3, pp. 278—287 (1970); Traynelis et al., *Journal of the American Chemical Society,* Vol. 87, pp. 5710—5715 (1965); and Geibel et al, *Journal of the American Chemical Society,* Vol. 100, pp. 3575—3585 (1978). The compounds in these references are variously disclosed as being suitable as pharmaceuticals, pharmaceutical intermediates, herbicides, monomers, and resin fillers, but no activity is mentioned as insect repellents.

As to the invention, the terms "alkyl" and "alkenyl" include both straight and branched chain groups. In a preferred embodiment R is 2-methyl-1-propenyl, $—CH{=}C(CH_3)_2$. A preferred compound of this type is gamma-(3-pyridyl)-propyl-3-methyl-2-butenoate (R=2-methyl-1-propenyl, and the ester moiety is substituted at the 3-position on the pyridyl ring). Other compounds include the 2-pyridyl and 4-pyridyl isomers of this preferred compound and gamma-(3-pyridyl)-propyl isovalerate (R=isobutyl, substitution at the 3-position on the ring).

The compounds of this type can be prepared by reaction of an appropriate pyridyl alkanol with an acyl chloride:

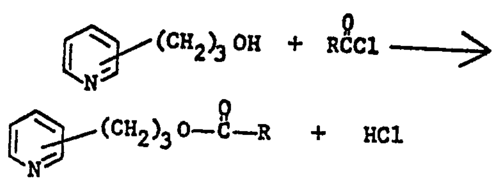

The reaction is generally conducted at temperatures of about 5—15°C, in the presence of a solvent such as methylene chloride, chloroform, other chloro-carbon solvents or ethers, and a hydrogen chloride acceptor. For preparation of compounds in which R is alkyl, suitable hydrogen chloride acceptors include pyridine, triethylamine and sodium hydroxide. For compounds in which R is alkenyl, pyridine is used. The product is recovered by conventional extraction, washing and filtration steps.

Preparation of such compounds is illustrated by the following example.

### Example 1

*Preparation of Gamma-(3-Pyridyl)-Propyl-3-Methyl-2-Butenoate*

Into a flask was introduced a solution of 5.00 grams (0.036 mole) of 3-(3-pyridyl)-1-propanol and 3.16 grams (0.040 mole) pyridine (reagent grade) in 50 milliliters of methylene chloride (reagent grade). A clear brown solution was formed which was then cooled to 0°C. with an ice-water bath. There was then added 4.74 grams (0.040 mole) of 3,3-dimethylacryloyl chloride (97% pure), dropwise, over a period of 10 minutes, the temperature being maintained between 5 and 15°C. The solution became deep red. Following the addition, the solution was stirred at 0°C for one hour; the cooling bath was removed and the solution stirred at room temperature for three additional hours.

The solution was then diluted with 50 milliliters of methylene chloride and poured into 50 milliliters of water; the organic layer was separated and washed with 10 milliliters of a saturated sodium bicarbonate solution and three portions each of 10 milliliters of water until the pH of the solution was measured at 7, followed by washing with 10 milliliters of sodium chloride and drying over sodium sulfate. The dried organic solution was filtered through sodium sulfate and the solvent was removed under vacuum to yield 8.08 grams (101.3% of theoretical) of the desired compound, a crude clear orange oil, $n_D^{30}$ 1.5153. The structure of the compound was confirmed by infrared and nuclear magnetic resonance spectra.

*Insect Repellent Test*

The compound prepared according to Example 1 was tested for insect repellency by the following procedures:

*Mosquito Repellent Evaluation:* A paper cup filled with pupae of the mosquito *Culex pipiens quinquefasciatus* (Say) was placed in a screen cage and the pupae allowed to emerge into adults. Sugar cubes were then saturated with 0.8 milliliters of acetone containing the test compound and, for a control, with the same amount of acetone alone. Concentrations of the test compound were utilized beginning at 1% and proceeding to lower concentrations. After the cubes dried, they were put into the screen cage. Repellency was determined by the number of mosquito adults lighting and feeding on the sugar cubes, with observations being made daily. New adult mosquitos were periodically added to the cages until all sugar cubes became non-repellent. The number of days of complete repellency of mosquitos from the sugar cubes were recorded. Comparative tests were similarly conducted using the compound N,N-diethyl-m-toluamide, commercially manufactured and employed as an insect repellent, and generally known by the generic name "deet". The results of the tests of deet and the compound of Example 1 are shown in the following Table I, the numbers of each column representing the number of days of complete repellency observed using the specified concentration.

Table I

| | CONCENTRATION, % | | | | |
|---|---|---|---|---|---|
| | 1 | 0.5 | 0.3 | 0.1 | 0 (acetone only) |
| Example 1 | > 60 | 27 | 26 | 5 | 0 |
| deet | 11—13 | 6.7 | 5 | 5 | 0 |

Thus, at a concentration as low as 0.1%, the new compound was as effective as deet in repelling mosquitos. At high concentrations, it was more effective than deet. At a concentration of 1% of the new compound in the solution, after 60 days the mosquitos were still repelled from the sugar cube and the tests were terminated.

*Houseflies:* The insect utilized for this test was housefly, Musca domestica (L.) One hundred (100) houseflies of mixed sexes were placed in test cages. Each test cage consisted of a 454 ml cup, covered with tulle netting, and having two 21 ml cups stapled on opposite sides of the upper, interior perimeter. One of the small cups contained a sugar cube saturated with 0.8 milliliters of acetone containing a specific concentration of the test compound. The cube was dried and weighed before being placed in the cup. The other small cup contained a water-saturated cotton plug. The test cages were placed on a turntable and rotated at 1.5 revolutions per minute to keep the flies randomly distributed inside the cages. After 72 hours the flies in each cage were anesthetized with carbon dioxide. The sugar cubes were removed and re-weighed and the percentage weight loss (due to consumption by the flies) recorded. The cubes with the least weight loss are considered to show the greatest repellency. Test concentrations of the compound ranged from 1% down to 0.1%. The weight losses of the sugar in percent, is shown in the following Table II.

TABLE II

| | CONCENTRATION, % | | | | |
|---|---|---|---|---|---|
| | 1 | 0.5 | 0.3 | 0.1 | 0 (Acetone only) |
| Example 1 | 4.45 | 5.91 | 6.73 | 9.5 | 12.7 |
| deet | 7.09 | 7.12 | 9.69 | 11.0 | 12.7 |

The novel compounds of this invention may be used as insect repellents in diluted or undiluted form. When used in a diluted form, the compounds may be embodied into compositions containing a relatively high or relatively low concentration of the active compound. For example, the active compound can be incorporated into relatively high concentration compositions such as wet sprays or

solutions in alcohol or other suitable solvents. Such compositions may contain, in addition to the active compound, adjuvants such as emulsifying agents, surface active agents, antioxidants, and propellants, which may be found normally in insect repellant preparations. The active compounds of this invention may be employed as the sole active component of such compositions or can be used in admixture with other compounds having similar or different utility. For example, the novel compounds may be incorporated into creams, lotions, powders, suntan oils, insecticides and other preparations, which may contain pesticidal or other useful substances, as well as to compositions of various types used for treating fabrics or articles of clothing to render them insect repellent. In general, compositions for repellent use may contain from 0.5 to as high as 80 weight %, preferably from 2 to about 40 weight %, of the novel compound.

**Claims**

1. A compound having the formula

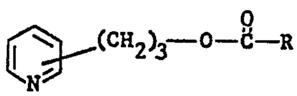

in which R is $C_3$—$C_5$ alkyl or $C_3$—$C_5$ alkenyl.

2. A compound according to Claim 1 in which R is 2-methyl-1-propenyl and the ester moiety is substituted at the 3-position on the pyridine ring.

3. An insect repellent composition of matter containing an amount of a compound having the formula

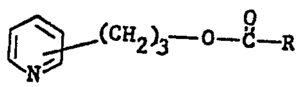

in which R is $C_3$—$C_5$ alkyl or $C_3$—$C_5$ alkenyl, effective to repel insects, and an inert diluent or carrier.

4. A composition according to Claim 3 containing an amount of a compound effective to repel mosquitos.

5. A composition according to Claim 3 in which R is 2-methyl-1-propenyl and the ester moiety is substituted at the 3-position on the pyridine ring.

6. A method of repelling insects from a locus to be protected therefrom, comprising applying to said locus an effective insect repelling amount of a compound according to claim 1.

7. A method according to Claim 6 in which the compound is applied in an amount effective to repel mosquitos.

8. A method according to Claim 6 in which R is 2-methyl-1-propenyl and the ester moiety is substituted at the 3-position on the pyridine ring.

**Revendications**

1. Composé présentant la formule:

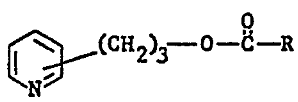

dans laquelle:

R est un radical alkyl en $C_3$—$C_5$ ou alkényl en $C_3$—$C_5$.

2. Composé selon la revendication 1, dans laquelle:

R est un radical 2-méthyl-1-propényl et la partie ester est substituée en position 3 du noyau pyridinique.

3. Composition pour repousser les insectes contenant une quantité efficace pour repousser les insectes d'un composé présentant la formule:

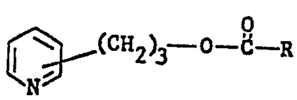

dans laquelle:

R est un radical alkyl en $C_3$—$C_5$ ou alkényl en $C_3$—$C_5$; et d'un diluant ou support inerte.

4. Composition selon la revendication 3, contenant une quantité d'un composé efficace pour repousser les moustiques.

5. Composition selon la revendication 3, dans laquelle:

R est un radical 2-méthyl-1-propényl et la partie ester est substituée en position 3 du noyau pyridinique.

6. Procédé pour repousser les insectes d'un lieu dont on vent les protéger comprenant l'application audit lieu d'une quantité efficace pour repousser les insectes d'un composé selon la revendication 1.

7. Procédé selon la revendication 6, dans lequel le composé est appliqué en une quantité efficace pour repousser les moustiques.

8. Procédé selon la revendication 6, dans lequel:

R est un radical 2-méthyl-1-propényl et la partie ester est substituée en position 3 du noyau pyridinique.

**Patentansprüche**

1. Chemische Substanz der Formel

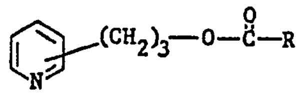

in der R ein Alkylrest mit 3 bis 5 Kohlenstoffatomen oder ein Alkenylrest mit 3 bis 5 Kohlenstoffatomen ist.

2. Substanz nach Anspruch 1, dadurch gekennzeichnet, daß R die 2-Methyl-1-propenylgruppe ist und der Esterteil in 3-Stellung des Pyridinringes substituiert ist.

3. Insektenabwehrmittel, enthaltend in einer zur Abwehr von Insekten wirksamen Menge eine chemische Substanz der Formel

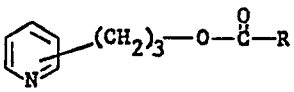

in der R eine Alkylgruppe mit drei bis fünf Kohlenstoffatomen oder eine Alkenylgruppe mit 3 bis 5 Kohlenstoffatomen ist, und ein inertes Verdünnungsmittel oder einen inerten Träger.

4. Mittel nach Anspruch 3, gekennzeichnet durch den Gehalt der Substanz in einer zur Abwehr von Moskitos wirksamen Menge.

5. Mittel nach Anspruch 3, dadurch gekennzeichnet, daß R die 2-Methyl-1-propenylgruppe ist und der Esterteil in 3-Stellung des Pyridinringes substituiert ist.

6. Verfahren zur Abwehr von Insekten von einem vor Insekten zu schützenden Ort, dadurch gekennzeichnet, daß der vor den Insekten zu schützende Ort mit einer Substanz gemäß Anspruch 1 in einer zur Abwehr von Insekten wirksamen Menge belegt wird.

7. Verfahren nach Anspruch 6, dadurch gekennzeichnet, daß die Substanz in einer zur Abwehr von Moskitos wirksamen Menge eingesetzt wird.

8. Verfahren nach Anspruch 6, dadurch gekennzeichnet, daß R die 2-Methyl-1-propenylgruppe ist und der Esterteil in 3-Stellung des Pyridinringes substituiert ist.